# FASCICULE DE BREVET EUROPEEN

(11) **EP 4 132 390 B1**
(45) Date de publication et mention de la délivrance du brevet: **21.02.2024**
(21) Numéro de dépôt: 21719066.9
(22) Date de dépôt: 08.04.2021
(51) Int. Cl.: A61B 17/68, A61B 17/84, A61C 8/00, A61B 17/86

(54) **IMPLANT D'ANCRAGE OSSEUX À EXTRACTION FACILITÉE**
LEICHT ENTFERNBARES KNOCHENVERANKERUNGSIMPLANTAT
EASY-TO-EXTRACT BONE ANCHORING IMPLANT

(30) Priorité: 09.04.2020 FR 2003581
(43) Date de publication de la demande: 15.02.2023
(73) Titulaire: Lock-In SA, 1180 Rolle (CH)
(72) Inventeur: LACAZE, Guillaume, 1278 LA RIPPE (CH)
(74) Mandataire: Debay, Damien
(86) Numéro de dépôt international: PCT/EP2021/059197
(87) Numéro de publication internationale: WO 2021/204953

(56) Documents cités:
- WO-A1-2012/045787
- US-A1- 2012 265 258
- US-A1- 2018 071 002
- US-A1- 2019 350 630
- US-B1- 8 388 660

## Description

### DOMAINE TECHNIQUE ET OBJET DE L'INVENTION

La présente invention se rapporte au domaine des implants osseux destinés à des applications dentaires, orthopédiques, chirurgicales ou ostéoplastiques, tels que des vis orthopédiques seules ou avec des plaques, des implants dentaires ou ligamentaires pour des articulations tels que par exemple les hanches, les coudes, les chevilles, les épaules et les genoux, ou spinaux rachidiens par exemple pour les vertèbres. Ces domaines d'application sont donnés à titre d'exemple et ne sont pas restrictifs quant à la portée de la présente invention.

Plus spécifiquement l'invention concerne un implant osseux dont l'implantation dans l'os poreux est extrêmement stable.

### ETAT DE LA TECHNIQUE

Un implant d'ancrage osseux est généralement constitué d'un corps de forme allongée destiné à être implanté dans un logement formé dans un tissu osseux, tel que l'os de la mâchoire pour une application dentaire ou dans une vertèbre par exemple. Il est important que l'implant d'ancrage osseux puisse être introduit facilement dans le tissu osseux, sans créer de dommages, et que le dispositif d'ancrage à l'intérieur du tissu osseux soit stable. En effet, les dispositifs d'implant d'ancrage osseux actuels ne permettent pas un ancrage sans engendrer des fissures ou des dommages plus importants que ne le nécessite la taille du dispositif lui-même dans le tissu osseux, de plus il est nécessaire que la fixation dans l'implant osseux soit fiable et extrêmement stable, car de nombreuses techniques thérapeutiques reposent aujourd'hui sur la croissance osseuse qui nécessite généralement que les dispositifs ancrés dans le tissu osseux restent le plus immobile possible.

En outre, il est aussi nécessaire que l'implantation dans le tissu osseux soit facile à réaliser pour éviter tout risque de mauvais positionnement de l'implant d'ancrage osseux, qui pourrait notamment être dû à une difficulté de positionnement ou d'implantation dans l'os.

De plus, en cas de chute, de choc ou d'accident, il est important que l'implant d'ancrage osseux reste en place dans le tissu osseux, c'est-à-dire qu'il ne se déplace pas à travers l'os. Pour cela, une très forte stabilité de l'implant est nécessaire.

Il est également important que le retrait de l'implant du tissu osseux où il est implanté soit possible et facilement réalisable, sans créer de blessures ou de dommages dans le tissu osseux.

L'état de la technique comprend le document WO 2012/045787 A1 qui divulgue un implant d'ancrage osseux comprenant un élément d'accrochage dans le tissu osseux lequel comprend une cavité avec un taraudage pour recevoir et coopérer avec un élément d'expansion fileté. L'élément d'accrochage comporte en outre des fentes longitudinales débouchantes aptes à permettre, en plus des propriétés élastiques intrinsèques au matériau de la partie d'accrochage, un élargissement encore plus important de la partie d'accrochage.

L'état de la technique comprend également le document de brevet EP2603163 B1, qui décrit un implant endo-osseux à ancrage amélioré apte à être implanté dans un tissu osseux et comportant un dispositif de fixation comprenant une partie dite d'accrochage dans le tissu osseux, et une partie dite d'expansion, ces deux parties étant mobiles l'une par rapport à l'autre. L'invention mentionnée dans ce brevet comprend également des moyens de liaison mécanique coopérants, disposés d'une part sur la partie d'accrochage et d'autre part sur la partie d'expansion, tels que la mobilité relative des deux parties comprend au moins un degré de liberté et tels qu'un déplacement relatif desdites deux parties provoque un élargissement de la partie d'accrochage, ledit élargissement provoquant l'accrochage de la partie d'accrochage dans le tissu osseux. L'implant osseux décrit dans ce brevet trouve particulièrement application dans le domaine dentaire.

Cependant, une telle solution présente des inconvénients car l'implant osseux bien qu'il soit immobilisé en rotation et en translation dans le tissu, présente le risque de bouger, notamment de reculer lors d'un choc, rendant par la suite son retrait moins aisé.

L'invention vise donc à résoudre ces inconvénients en proposant un implant d'ancrage osseux apte à être implanté et immobilisé dans le tissu osseux de façon extrêmement stable, puis à être dévissé et retiré du tissu osseux si nécessaire, sans créer de lésions dans le tissu osseux.

### PRESENTATION GENERALE DE L'INVENTION

La présente invention a donc pour objet de pallier les inconvénients de l'art antérieur en proposant un implant d'ancrage osseux, ci-après dénommé implant osseux ; facilement implantable dans le tissu osseux, stable et qui puisse également être retiré facilement du tissu osseux.

Pour parvenir à ce résultat, la présente invention concerne un implant d'ancrage osseux à extraction facilitée comprenant :
Un manchon expansible s'étendant entre une portion proximale possédant un premier diamètre intérieur, et une portion distale possédant un second diamètre intérieur inférieur audit premier diamètre intérieur, ces deux portions définissant un axe longitudinal (L) et lesdits premier et second diamètres intérieur définissant un profil intérieur dudit manchon expansible, et comprenant, d'une part, au moins un premier filetage à l'intérieur du manchon expansible et, d'autre part, au moins un second filetage à l'extérieur du manchon expansible,
Une vis s'étendant entre une portion proximale et une portion distale sur un axe colinéaire à l'axe (L) et présentant, d'une part, le long dudit axe longitudinal (L), un profil extérieur complémentaire audit profil intérieur dudit manchon expansible et, d'autre part, au moins un filetage extérieur dont le pas de vis est inversé par rapport audit second filetage extérieur du manchon expansible,
L'implant étant apte à passer d'une position repliée de repos à une position déployée par l'actionnement desdits filetages inversés en provoquant la pénétration de la vis dans le manchon expansible et engendrant l'expansion dudit manchon expansible par déformation, grâce au fait que le diamètre extérieur de la vis est supérieur, au moins sur une portion distale, audit second diamètre intérieur du manchon expansible,
En position déployée de l'implant, le second diamètre intérieur du manchon expansible étant supérieur ou égal au premier diamètre intérieur,
La portion distale comportant une tête comprenant au moins une goujure arrière à bord coupant pour fraiser l'os lors de l'extraction de l'implant osseux et faciliter le retrait de l'implant.

Selon une particularité, le bord coupant de ladite goujure arrière présente un angle, par rapport à l'axe longitudinal (L), déterminé par le sens de filetage de la vis lors du retrait de l'implant.

Selon une autre particularité, le diamètre extérieur de la vis est supérieur au diamètre intérieur du manchon expansible, par au moins un rétrécissement sur une portion distale.

Selon une autre particularité, ledit au moins un rétrécissement est situé, par rapport à la portion proximale et selon l'axe longitudinal (L), à une distance déterminée en fonction de l'épaisseur de la profondeur, dans le tissu osseux, à laquelle ladite expansion est souhaitée.

Selon une autre particularité, l'implant comprend un filetage externe d'ancrage osseux sur une portion tronconique dont l'évasement est inversé par rapport à l'évasement formé par le tronc de cône du manchon en position déployée.

Selon une autre particularité, ledit manchon expansible comporte des fentes longitudinales débouchantes s'étendant jusqu'à sa portion distale.

Selon une autre particularité, la portion distale du manchon est auto-taraudante grâce au fait qu'elle comporte au moins une encoche ou une goujure.

Selon une autre particularité, il y a autant d'encoches ou de goujures auto-taraudantes que de fentes longitudinales débouchantes.

Selon une autre particularité, ledit manchon expansible comporte des fentes longitudinales non débouchantes.

Selon une autre particularité, ladite vis comprend au moins un marqueur de distance pour visualiser le moment où le vissage de la vis dans le manchon expansible doit être réalisé dans le sens opposé au vissage du manchon expansible dans le tissu osseux.

Selon une autre particularité, ledit manchon expansible est réalisé en matière plastique ou élastique.

### PRESENTATION DES FIGURES

D'autres caractéristiques et avantages de l'invention apparaîtront à la lecture de la description détaillée des modes de réalisation de l'invention, donnés à titre d'exemple uniquement, et en référence aux dessins qui montrent :
[Fig. 1a], [Fig. 1b] et [Fig. 2] représentent une vue détaillée des éléments qui composent l'implant osseux selon l'invention.
[Fig. 3a] représente un schéma du manchon de l'implant osseux avant l'expansion selon l'invention.
[Fig. 3b] représente un schéma du manchon de l'implant osseux après l'expansion selon l'invention.
[Fig. 3c] représente un schéma de l'implant osseux après l'expansion selon l'invention.
[Fig. 4a] et [Fig. 4b] représentent une vue de l'intérieur de l'implant osseux avant l'expansion du manchon selon l'invention.
[Fig. 5] et [Fig. 6] représentent un schéma de l'implant osseux après l'expansion du manchon selon l'invention.
[Fig. 7] et [Fig. 8] représentent un schéma du manchon en position expandue selon l'invention.

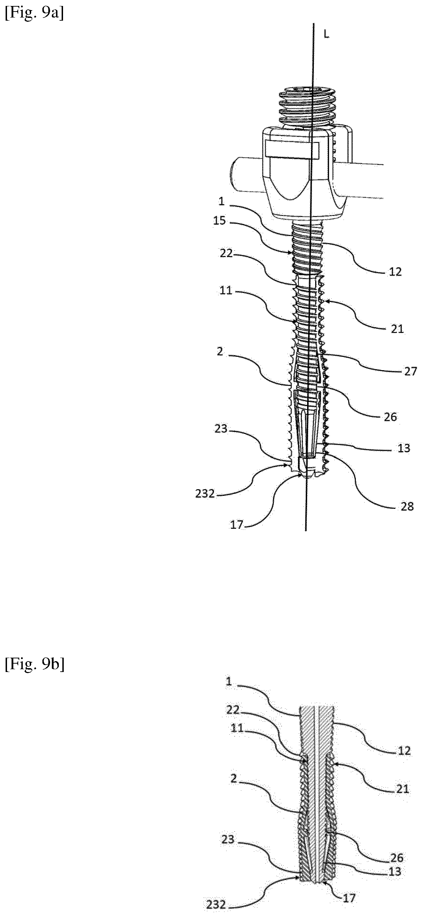

[Fig. 9a] et [Fig. 9b] représentent un schéma de l'intérieur de l'implant osseux après l'expansion du manchon selon l'invention.

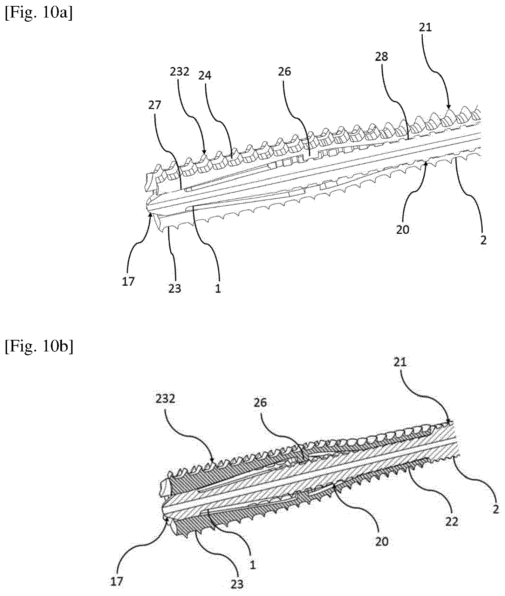

[Fig. 10a] et [Fig. 10b] représentent un schéma de l'intérieur du manchon en position expandue selon l'invention.
[Fig. 11] représente un schéma d'une coupe transversale de l'intérieur de l'implant osseux, avant l'expansion selon l'invention.
[Fig. 12] représente un schéma de la pointe de vis, selon l'invention.

### DESCRIPTION DETAILLE D'UNE FORME DE REALISATION DE L'INVENTION

Divers modes de réalisation de l'invention sont décrits ci-dessous notamment en référence aux figures illustratives et non limitatives.

La présente demande concerne l'extraction d'un implant osseux du tissu osseux.

Il peut arriver que la présence d'un implant osseux ne soit plus nécessaire dans le tissu osseux ou qu'il soit nécessaire d'ôter cet implant osseux.

On notera ici que l'on désigne par le terme « extraction » le fait de retirer l'implant osseux du tissu osseux, généralement par dévissage, si cela est nécessaire. L'extraction proposée dans la présente demande désigne un retrait de cet implant osseux du tissu osseux en limitant les dommages liés à ce retrait, lorsqu'une ablation est souhaitée.

Par ailleurs, le terme « tissu(s) osseux » désigne généralement tous types d'os, qu'il s'agisse d'os compact (comme par exemple l'os cortical ou le périoste) ou d'os spongieux (mou, poreux), car le système d'implant osseux de la présente demande est implantable dans tout type de tissu osseux.

En outre, les termes utilisés ne doivent pas être interpréter dans leur acceptation générale mais plutôt à la lumière des considérations fonctionnelles détaillées dans la présente demande.

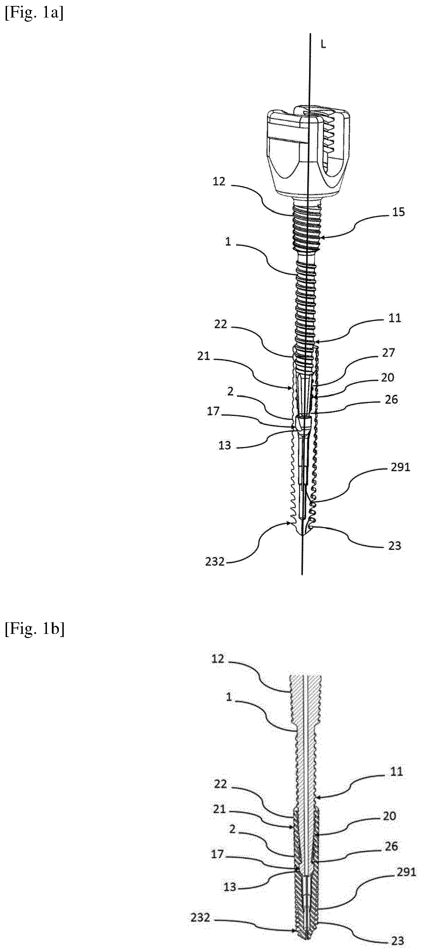

[Fig. 1a] et [Fig. 1b] sont un exemple de réalisation illustratif et non limitatif de l'implant osseux.

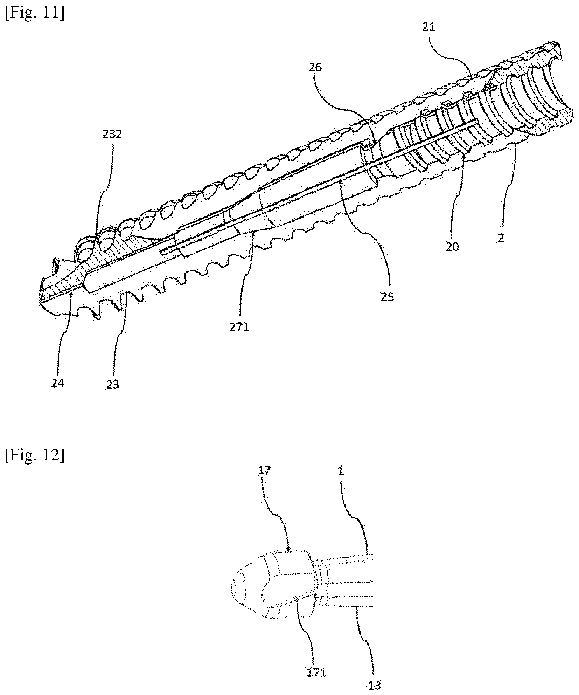

Comme par exemple représenté sur [Fig. 1a] à [Fig. 11], un implant osseux apte à être implanté dans un tissu osseux comprend un manchon expansible (2) s'étendant entre une portion proximale (22) possédant un premier diamètre, et une portion distale (23) possédant un second diamètre, ces deux portions définissant un axe longitudinal (L), et comprenant, d'une part, au moins un premier filetage (20) à l'intérieur du manchon expansible (2) et, d'autre part, au moins un second filetage (21) à l'extérieur du manchon expansible (2), ledit manchon expansible (2) comportant des fentes longitudinales débouchantes (24) s'étendant jusqu'à sa portion distale (23) et des encoches (231) auto-taraudantes, ainsi que des fentes longitudinales non débouchantes (25).

Dans la présente demande, le terme manchon expansible (2) désigne généralement un cylindre généralisé (generalized cylinder en anglais) creux.

Dans certains modes de réalisation, l'implant osseux comprend aussi une vis (1) s'étendant entre une portion proximale (12) et une portion distale (13) sur un axe colinéaire à l'axe (L) et présentant, d'une part, le long dudit axe longitudinal (L), un profil extérieur complémentaire au profil intérieur dudit manchon expansible (2) et, d'autre part, au moins un filetage (11) extérieur dont le pas de vis est inversé par rapport audit second filetage extérieur (21) du manchon expansible (2), ladite vis (1) comprenant au moins un marqueur de distance (16) pour visualiser le moment où le vissage de la vis (1) dans le manchon expansible (2) doit être réalisé dans le sens opposé au vissage du manchon expansible (2) dans le tissu osseux.

Les termes « proximal » et « distal » désignent dans la présente demande, respectivement, la partie où le dispositif d'implantation est tenu pour permettre son implantation dans le tissu osseux, et la partie qui est implantée en premier dans le tissu osseux (à l'opposé de la portion proximale).

Les termes « portions » proximale et distale désignent dans la présente demande les parties situées à proximité des extrémités distales et proximales.

Il est à noter que la portion proximale (12) de la vis (1) est directement implantée dans l'os cortical.

Il est aussi à noter que l'implant osseux est réalisé en titane ou en acier inoxydable médical implantable ou en polyétheréthercétone (PEEK) ou en polyéthercétonecétone (PEKK) ou en tout autre matériau dont l'homme du métier pourra déterminer l'adéquation en fonction de ses propriétés mécaniques, physicochimiques et de sa biocompatibilité.

Dans certains modes de réalisation, l'implant est apte à passer d'une position repliée de repos à une position déployée par l'actionnement desdits filetages inversés en provoquant la pénétration de la vis (1) dans le manchon expansible (2) et engendrant l'expansion dudit manchon expansible (2) par déformation, grâce au fait que le diamètre extérieur de la vis (1) est supérieur au diamètre intérieur du manchon expansible (2), au moins sur une portion distale. En position déployée de l'implant, le second diamètre du manchon expansible (2) est supérieur ou égal au premier diamètre.

Dans certains modes de réalisation, le diamètre extérieur de la vis (1) est supérieur au diamètre intérieur du manchon expansible, par au moins un rétrécissement (271) sur une portion distale.

Dans certains modes de réalisation, ledit au moins un rétrécissement (271) est situé, par rapport à la portion proximale et selon l'axe longitudinal (L), à une distance déterminée en fonction de l'épaisseur de la profondeur, dans le tissu osseux, à laquelle ladite expansion est souhaitée.

Dans certains modes de réalisation, l'extrémité proximale de la vis (1) comprend un moyen d'actionnement permettant de visser la vis (1), ledit moyen d'actionnement comprenant une structure de toute forme souhaitable par le praticien selon l'utilisation qui en sera faite, comme par exemple représenté sur [Fig. 1b]. Le moyen d'actionnement étant par exemple un trou à six pans ou un torx ou un cruciforme ou tout autre moyen d'actionnement, et l'extrémité proximale de la vis (1) pourra avoir diverses formes en fonction de la destination souhaitable pour l'implant d'ancrage osseux (tête de fixation de barre d'ostéosynthèse polyaxiale ou non, ou fixation de plaque ou tout autre dispositif).

Dans certains modes de réalisation, la vis (1) comprend une canule traversant la vis (1) pour permettre au praticien d'injecter par exemple du ciment, s'il estime cela nécessaire.

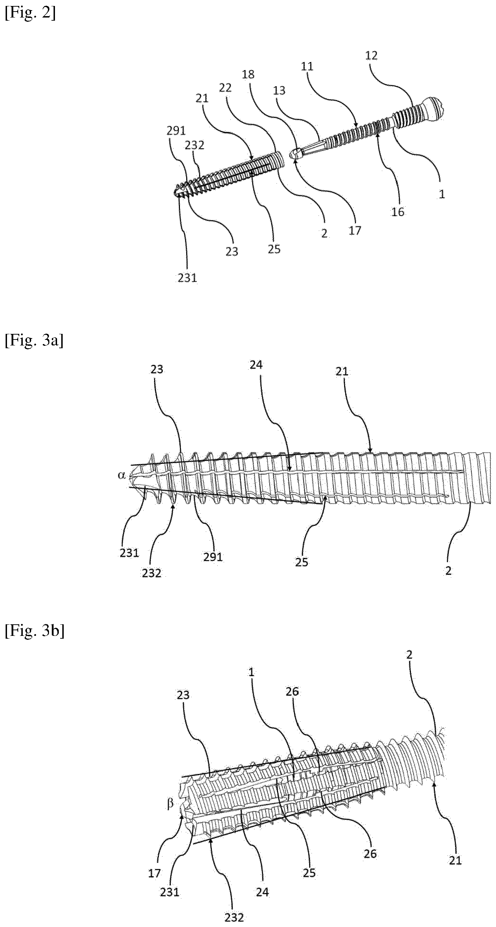

Dans certains modes de réalisation, l'implant comprend aussi un filetage externe (15) d'ancrage osseux sur une portion tronconique, comme cela est représenté par exemple sur [Fig. 1a], [Fig. 1b] et [Fig. 2].

Le terme « ancrage osseux » utilisé dans la présente demande désigne de façon générale divers types de dispositifs comprenant au moins un élément destiné à pénétrer le tissu osseux, selon un trajet rectiligne, sous l'action d'une poussée généralement exercée sous la forme de frappes, d'impacts, ou de vissages répétés. Il est connu qu'un filetage d'ancrage osseux possède une hauteur de filet généralement supérieure à celle d'un filetage mécanique pour assurer un meilleur ancrage. En outre, un filetage d'ancrage osseux est généralement différent d'un filetage mécanique et l'Homme du métier sait, qu'en fonction du type d'os et de l'application souhaitée, il est possible de faire varier le diamètre de l'âme, le pas de vis et la hauteur de fil et la présente demande couvre ces divers modes de réalisation.

Dans certains modes de réalisation, les profils extérieur de la vis (1) et intérieur du manchon expansible (2) sont complémentaires, de sorte qu'ils fournissent, en configuration expandue :
Un point d'appui proximal supporté par la complémentarité du diamètre externe de la vis (1) avec le diamètre interne du manchon expansible (2),
Un point d'appui distal supporté pas la coopération entre le manchon expansible (2) dont le diamètre interne se rétrécit vers la portion distale jusqu'à être inférieur au diamètre externe de la vis (1),
Un point d'appui « central » localisé entre ces deux points d'appuis, formé par la coopération entre le diamètre externe de la vis (1) et le diamètre interne du manchon expansible (2) qui induisent un diamètre externe du manchon expansible (2) au niveau « central » qui est supérieur au diamètre externe du manchon expansible (2) au niveau du point d'appui proximal.

Dans certains modes de réalisation, comme par exemple représenté sur [Fig. 3a], le manchon expansible (2) présente un angle aigu α à l'extrémité de sa portion distale (23). Cet angle α s'ouvre et augmente au fur et à mesure que la vis (1) pénètre dans le manchon expansible (2), lors de l'expansion.

Dans certains modes de réalisation, comme par exemple représenté sur [Fig. 3b], l'angle α en s'ouvrant de plus en plus lors de l'expansion devient un angle β, l'angle β étant l'angle du manchon expansible (2) expandu.

On notera qu'en position déployée, les parois du corps tubulaire (2) peuvent dans certains modes de réalisation être parallèles au lieu de créer un angle β.

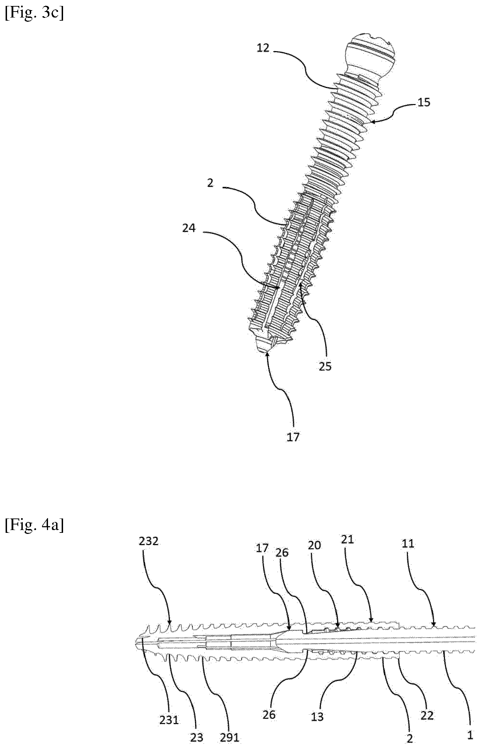

Dans certains modes de réalisation, le corps tubulaire (2) présente une forme bombée au niveau du point d'appui central, comme par exemple représenté sur [Fig. 3c], par la présence des angles α et β.

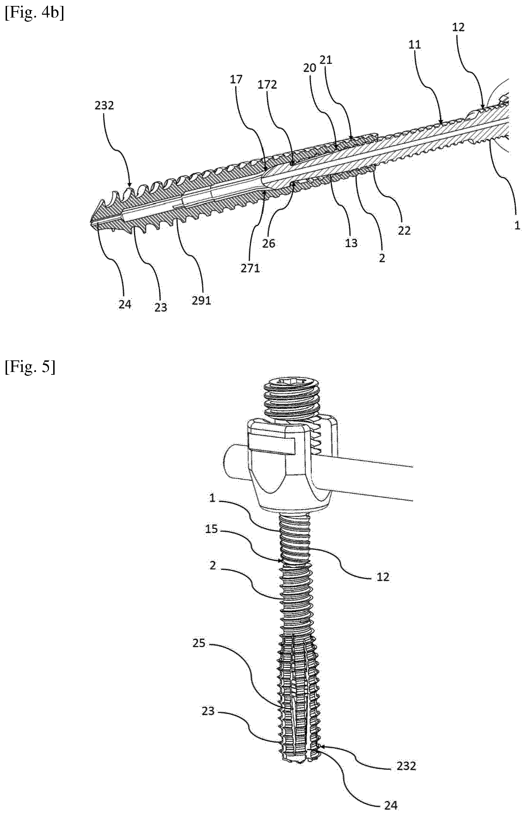

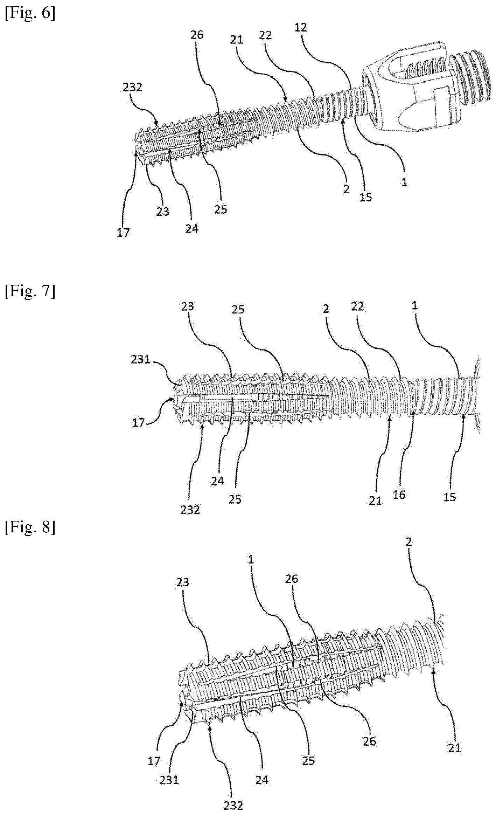

Dans certains modes de réalisation, comme par exemple représenté sur [Fig. 3a] à [Fig. 3c], [Fig. 5] à [Fig. 8], le manchon expansible (2) comporte des fentes longitudinales débouchantes (24) s'étendant jusqu'à sa portion distale (23) et des fentes longitudinales non débouchantes (25) permettant l'expansion du manchon expansible (2). Il est préférable qu'il y ait plusieurs fentes débouchantes (24) ou non débouchantes (25), et que la portion distale (23) comporte les deux types de fentes, c'est-à-dire des fentes longitudinales débouchantes (24) et des fentes longitudinales non débouchantes (25).

Dans certains modes de réalisation, la synergie entre les fentes débouchantes (24) et les fentes non débouchantes (25) permet en outre une géométrie de cône tronqué. Dans certains modes de réalisation, les fentes non débouchantes (25) permettent au corps tubulaire (2) de s'expandre dans le tissu osseux spongieux en présentant une forme bombée, de type convexe, permettant de comprimer et densifier la matière sur sa périphérie, améliorant ainsi la stabilité primaire, la cicatrisation, et permettant d'éviter l'ajout de ciment pour stabiliser et immobiliser l'implant osseux.

Dans certains modes de réalisation, il y a autant d'encoches (231) auto-taraudantes que de fentes longitudinales débouchantes (24).

Dans certains modes de réalisation, les fentes débouchantes (24) et les fentes non débouchantes (25) sont positionnées en décalage l'une par rapport à l'autre sur la longueur du manchon expansible (2). Le décalage des fentes débouchantes (24) et des fentes non débouchantes (25) sur la longueur améliore la souplesse et la résistance mécanique du manchon expansible (2) lors de l'expansion.

Dans certains modes de réalisation, les fentes longitudinales débouchantes (24) et non débouchantes (25) sur la portion distale (23) permettent l'expansion cylindrique du manchon expansible (2). Les fentes longitudinales non débouchantes (25) contribuent à la stabilité de l'implant osseux dans le tissu osseux en permettant lors de l'expansion de pouvoir conserver le profil de contact sur les trois points d'appui entre le manchon expansible (2) et la vis (1), et en laissant les efforts dus à l'expansion se répartir de façon homogène sur la périphérie du manchon expansible (2) expandu. Les fentes longitudinales débouchantes (24) et non débouchantes (25) permettent une expansion radiale de la portion proximale (22) du manchon expansible (2) en respectant la limite élastique de la matière du manchon expansible (2) et son rétreint élastique lors du dévissage.

Dans certains modes de réalisation, lesdites fentes longitudinales débouchantes (24) s'étendent sur 10 à 90 % de la longueur du manchon expansible (2).

Il est à noter qu'une croissance osseuse passe à travers les interstices à l'intérieur de l'implant osseux que sont les fentes débouchantes (24) et les fentes non débouchantes (25). La repousse d'os contribue à l'immobilisation et à la stabilité de l'implant dans le tissu osseux, mais rend également plus difficile le retrait de l'implant lorsqu'une ablation est nécessaire après plusieurs mois d'implantation dans le tissu osseux.

Dans certains modes de réalisation, le marqueur de distance (16) est réalisé au laser. Dans certains modes de réalisation, l'implant osseux est retiré du tissu osseux, sans générer de lésions graves ou de fissures importantes dans l'os grâce notamment à la déformation élastique du manchon expansible (2) dans la limite de l'élasticité du matériau utilisé.

Comme représenté par exemple sur [Fig. 12], la tête (17) comprend au moins une encoche du type goujure arrière (171) de fraisage de l'os, afin de fraiser ou tarauder l'os lors de l'extraction de l'implant osseux. En effet, après insertion de l'implant dans la partie spongieuse de l'os, le tissu osseux se reforme en devenant plus dur autour de l'implant et dans les interstices laissés vides dans les fentes qui se sont ouvertes et/ou entre la paroi interne du manchon expansible (2) et la paroi externe de la vis (1) après l'expansion. Il est alors nécessaire de fraiser ou tarauder la partie dure de l'os pour pouvoir bouger l'implant et le dévisser lors de l'extraction, afin de retirer l'implant osseux.

Dans certains modes de réalisation, un court vissage supplémentaire permet de casser le tissu osseux reformé autour de l'implant afin de débloquer ce dernier, suivi d'un dévissage qui permet ensuite de retirer l'implant.

Dans certains modes de réalisation, lors de l'ablation de l'implant osseux, les goujures arrière (171) permettent de fraiser ou de tarauder l'os qui s'est constitué entre la vis (1) et le manchon expansible (2), lors du recul de la vis (1) jusqu'au retour à la position de repos dans laquelle les diamètres extérieurs sont sensiblement constants sur toute la longueur du manchon expansible (2). Ce retour à diamètre constant étant automatique lorsque le manchon expansible (2) a une déformation élastique, alors que lorsque le manchon expansible (2) est à déformation plastique ce retour à diamètre constant sera imposé par la compression subie par le manchon expansible (2) au cours du retrait à la sortie du conduit créé par l'implant.

Dans certains modes de réalisation, les goujures arrières (171) présentent un bord coupant présentant un angle par rapport à l'axe longitudinal (L) qui est fonction du sens de filetage utilisé lors du retrait de l'implant, ledit angle étant adapté au sens de rotation du vissage.

L'extraction de l'implant osseux comprend les étapes suivantes:
Le dévissage de l'implant osseux dans le sens du filetage extérieur (11) jusqu'à l'apparition du marqueur de distance (16),
Le verrouillage de l'implant par une pince bloquant la vis (1) dans le manchon expansible (2) en position de repos,
Le dévissage de l'implant dans le sens du second filetage (21).

L'implant osseux proposé dans l'invention peut donc être extrait du tissu osseux rapidement et de façon précise, son retrait étant facilité et/ou les risques d'endommager l'os lors de son retrait étant limités.

La présente demande décrit diverses caractéristiques techniques et avantages en référence aux figures et/ou à divers modes de réalisation. L'homme de métier comprendra que les caractéristiques techniques d'un mode de réalisation donné peuvent en fait être combinées avec des caractéristiques d'un ou plusieurs autre(s) mode(s) de réalisation à moins que l'inverse ne soit explicitement mentionné ou que ces caractéristiques ne soient incompatibles ou que la combinaison ne fonctionne pas.

Plus généralement, des combinaisons de divers types de moyen de retenue de l'implant et/ou de moyen de retenue des épineuses sont envisagées et seront appréciées par l'homme de métier à l'aide des considérations fonctionnelles et structurelles fournies dans la présente demande. De plus, les caractéristiques techniques décrites dans un mode de réalisation donné peuvent être isolées des autres caractéristiques de ce mode à moins que l'inverse ne soit explicitement mentionné, notamment car les considérations fonctionnelles fournies dans la présente demande fourniront une explication suffisante pour que les adaptations structurelles éventuellement nécessaires soient à la portée de l'homme de métier.

Les personnes versées dans l'art, à la lecture de la présente demande, comprendront que des modes de réalisation sous de nombreuses autres formes spécifiques que celles décrites en détail sont possibles sans l'éloigner du domaine d'application de l'invention comme revendiqué. Par conséquent, les présents modes de réalisation doivent être considérés à titre d'illustration, mais peuvent être modifiés dans le domaine défini par la portée des revendications jointes, et l'invention ne doit être limitée aux détails donnés ci-dessus.

## Revendications

1. Implant d'ancrage osseux à extraction facilitée comprenant :
Un manchon expansible (2) s'étendant entre une portion proximale (22) possédant un premier diamètre intérieur, et une portion distale (23) possédant un second diamètre intérieur inférieur audit premier diamètre intérieur, ces deux portions définissant un axe longitudinal (L) et lesdits premier et second diamètres intérieur définissant un profil intérieur dudit manchon expansible (2), et comprenant, d'une part, au moins un premier filetage (20) à l'intérieur du manchon expansible (2) et, d'autre part, au moins un second filetage (21) à l'extérieur du manchon expansible (2),
Une vis (1) s'étendant entre une portion proximale (12) et une portion distale (13) sur un axe colinéaire à l'axe (L) et présentant, d'une part, le long dudit axe longitudinal (L), un profil extérieur complémentaire audit profil intérieur dudit manchon expansible (2) et, d'autre part, au moins un filetage (11) extérieur dont le pas de vis est inversé par rapport audit second filetage extérieur (21) du manchon expansible (2),
L'implant étant apte à passer d'une position repliée de repos à une position déployée par l'actionnement desdits filetages inversés en provoquant la pénétration de la vis (1) dans le manchon expansible (2) et engendrant l'expansion dudit manchon expansible (2) par déformation, grâce au fait que le diamètre extérieur de la vis (1) est supérieur, au moins sur une portion distale, audit second diamètre intérieur du manchon expansible (2),
En position déployée de l'implant, le second diamètre intérieur du manchon expansible (2) étant supérieur ou égal au premier diamètre intérieur,
La portion distale (13) comportant une tête (17) comprenant au moins une goujure arrière (171) à bord coupant
**caractérisé en ce que** ladite goujure arrière (171) à bord coupant est apte à fraiser l'os lors de l'extraction de l'implant osseux et faciliter le retrait de l'implant.

2. Implant selon la revendication 1, **caractérisé en ce que** le bord coupant de ladite goujure arrière (171) présente un angle, par rapport à l'axe longitudinal (L), déterminé par le sens de filetage de la vis (1) lors du retrait de l'implant.

3. Implant selon les revendications précédentes, **caractérisé en ce que** le diamètre extérieur de la vis (1) est supérieur au diamètre intérieur du manchon expansible (2), par au moins un rétrécissement (271) sur une portion distale.

4. Implant selon la revendication 3, **caractérisé en ce que** ledit au moins un rétrécissement (271) est situé, par rapport à la portion proximale et selon l'axe longitudinal (L), à une distance déterminée en fonction de la profondeur, dans le tissu osseux, à laquelle ladite expansion est souhaitée.

5. Implant selon les revendications 1 à 4, **caractérisé en ce que** l'implant comprend un filetage externe (15, 152) d'ancrage osseux sur une portion tronconique dont l'évasement est inversé par rapport à l'évasement formé par le tronc de cône du manchon (2) en position déployée.

6. Implant selon les revendications précédentes, **caractérisé en ce que** ledit manchon expansible (2) comporte des fentes longitudinales débouchantes (24) s'étendant jusqu'à sa portion distale (23).

7. Implant selon les revendications précédentes, **caractérisé en ce que** la portion distale (23) du manchon est auto-taraudante grâce au fait qu'elle comporte au moins une encoche ou une goujure (231).

8. Implant selon les revendications 6 et 7, **caractérisé en ce qu'**il y a autant d'encoches ou de goujures (231) auto-taraudantes que de fentes longitudinales débouchantes (24).

9. Implant selon les revendications précédentes, **caractérisé en ce que** ledit manchon expansible (2) comporte des fentes longitudinales non débouchantes (25).

10. Implant selon l'une quelconque des revendications précédentes, **caractérisé en ce que** ladite vis (1) comprend au moins un marqueur de distance (16) pour visualiser le moment où le vissage de la vis (1) dans le manchon expansible (2) doit être réalisé dans le sens opposé au vissage du manchon expansible (2) dans le tissu osseux.

11. Implant selon les revendications précédentes, **caractérisé en ce que** ledit manchon expansible (2) est réalisé en matière plastique ou élastique.

## Patentansprüche

1. Knochenverankerungsimplantat mit erleichterter Extraktion, umfassend:
eine erweiterungsfähige Hülse (2), die sich zwischen einem proximalen Abschnitt (22), der einen ersten Innendurchmesser besitzt, und einem distalen Abschnitt (23), der einen zweiten Innendurchmesser besitzt, der kleiner als der erste Innendurchmesser ist, erstreckt, wobei diese zwei Abschnitte eine Längsachse (L) definieren und der erste und der zweite Innendurchmesser ein Innenprofil der erweiterungsfähigen Hülse (2) definieren und einerseits mindestens ein erstes Gewinde (20) im Inneren der erweiterungsfähigen Hülse (2) und andererseits mindestens ein zweites Gewinde (21) außerhalb der erweiterungsfähigen Hülse (2) umfassen,
eine Schraube (1), die sich zwischen einem proximalen Abschnitt (12) und einem distalen Abschnitt (13) auf einer Achse, die kollinear zu der Achse (L) verläuft, erstreckt, und die einerseits entlang der Längsachse (L) ein Außenprofil, das komplementär zu dem Innenprofil der erweiterungsfähigen Hülse (2) ist, und andererseits mindestens ein Außengewinde (11) aufweist, dessen Gewindesteigung in Bezug auf das zweite Außengewinde (21) der erweiterungsfähigen Hülse (2) umgekehrt ist,
wobei das Implantat geeignet ist, sich durch Betätigen der umgekehrten Gewinde bei Verursachen der Eindringung der Schraube (1) in die erweiterungsfähige Hülse (2) und Schaffen der Erweiterung der erweiterungsfähigen Hülse (2) durch Verformung, dank der Tatsache, dass der Außendurchmesser der Schraube (1) mindestens an einem distalen Abschnitt größer als der zweite Innendurchmesser der erweiterungsfähigen Hülse (2) ist, von einer eingeklappten Ruhestellung in eine ausgeklappte Stellung zu bewegen,
wobei in der ausgeklappten Position des Implantats der zweite Innendurchmesser der erweiterungsfähigen Hülse (2) größer oder gleich dem ersten Innendurchmesser ist, **dadurch gekennzeichnet, dass**
der distale Abschnitt (13) einen Kopf (17), umfassend mindestens eine hintere Nut (171) mit Schneidkante, vorweist, um bei Extraktion des Knochenimplantats den Knochen zu fräsen und das Entfernen des Implantats zu erleichtern.

2. Implantat nach Anspruch 1, **dadurch gekennzeichnet, dass** die Schneidkante der hinteren Nut (171) einen Winkel in Bezug auf die Längsachse (L) aufweist, der durch die Gewinderichtung der Schraube (1) bei Entfernen des Implantats bestimmt wird.

3. Implantat nach den vorstehenden Ansprüchen, **dadurch gekennzeichnet, dass** der Außendurchmesser der Schraube (1) durch mindestens eine Verengung (271) an einem distalen Abschnitt größer als der Innendurchmesser der erweiterungsfähigen Hülse (2) ist.

4. Implantat nach Anspruch 3, **dadurch gekennzeichnet, dass** die mindestens eine Verengung (271) in Bezug auf den proximalen Abschnitt und entlang der Längsachse (L) in einem Abstand angeordnet ist, der in Abhängigkeit von der Tiefe in dem Knochengewebe, bis zu der die Erweiterung gewünscht wird, bestimmt wird.

5. Implantat nach Anspruch 1 bis 4, **dadurch gekennzeichnet, dass** das Implantat ein Außengewinde (15, 152) für die Knochenverankerung an einem kegelstumpfförmigen Abschnitt umfasst, dessen Aufweitung in Bezug auf die Aufweitung, die durch den Kegelstumpf der Hülse (2) in der ausgeklappten Position ausgebildet wird, umgekehrt ist.

6. Implantat nach den vorstehenden Ansprüchen, **dadurch gekennzeichnet, dass** die erweiterungsfähige Hülse (2) durchgehende Längsschlitze (24) vorweist, die sich bis zu ihrem distalen Abschnitt (23) erstrecken.

7. Implantat nach den vorstehenden Ansprüchen, **dadurch gekennzeichnet, dass** der distale Abschnitt (23) der Hülse, dank der Tatsache, dass er mindestens eine Kerbe oder eine Nut (231) vorweist, selbstschneidend ist.

8. Implantat nach Anspruch 6 und 7, **dadurch gekennzeichnet, dass** es ebenso viele selbstschneidende Kerben oder Nuten (231) wie durchgehende Längsschlitze (24) gibt.

9. Implantat nach den vorstehenden Ansprüchen, **dadurch gekennzeichnet, dass** die erweiterungsfähige Hülse (2) nicht durchgehende Längsschlitze (25) vorweist.

10. Implantat nach einem der vorstehenden Ansprüche, **dadurch gekennzeichnet, dass** die Schraube (1) mindestens eine Abstandsmarkierung (16) zum Visualisieren des Moments, zu dem das Eindrehen der Schraube (1) in die erweiterungsfähige Hülse (2) in der Richtung entgegengesetzt zu dem Eindrehen der erweiterungsfähigen Hülse (2) in das Knochengewebe erfolgen muss, umfasst.

11. Implantat nach den vorstehenden Ansprüchen, **dadurch gekennzeichnet, dass** die erweiterungsfähige Hülse (2) aus Kunststoffmaterial oder elastischem Material hergestellt ist.

## Claims

1. An osseous anchoring implant with facilitated extraction, comprising:
An expandable sleeve (2) extending between a proximal portion (22) having a first internal diameter, and a distal portion (23) having a second internal diameter smaller than said first internal diameter, these two portions defining a longitudinal axis (L) and said first and second internal diameters defining an internal profile of said expandable sleeve (2), and comprising, on the one hand, at least a first threading (20) inside the expandable sleeve (2) and, on the other hand, at least a second threading (21) outside the expandable sleeve (2),
A screw (1) extending between a proximal portion (12) and a distal portion (13) on an axis collinear with the axis (L) and having, on the one hand, along said longitudinal axis (L), an external profile complementary to said internal profile of said expandable sleeve (2) and, on the other hand, at least one external threading (11) whose screw pitch is reversed relative to said second external threading (21) of the expandable sleeve (2),
The implant being able to switch from a folded rest position to a deployed position by the actuation of said reversed threadings by causing the penetration of the screw (1) into the expandable sleeve (2) and generating the expansion of said expandable sleeve (2) by deformation, thanks to the fact that the external diameter of the screw (1) is greater, at least on a distal portion, than said second internal diameter of the expandable sleeve (2),
In the deployed position of the implant, the second internal diameter of the expandable sleeve (2) being greater than or equal to the first internal diameter,
**Characterized in that**
The distal portion (13) includes a head (17) comprising at least one rear flute (171) with a cutting edge to mill the bone during the extraction of the osseous implant and facilitate the removal of the implant.

2. The implant according to claim 1, **characterized in that** the cutting edge of said rear flute (171) has an angle, relative to the longitudinal axis (L), determined by the thread direction of the screw (1) when the implant is removed.

3. The implant according to the preceding claims, **characterized in that** the external diameter of the screw (1) is greater than the internal diameter of the expandable sleeve (2), by at least one shrinkage (271) on a distal portion.

4. The implant according to claim 3, **characterized in that** said at least one shrinkage (271) is located, relative to the proximal portion and along the longitudinal axis (L), at a distance determined as a function of the depth, in the osseous tissue, at which said expansion is desired.

5. The implant according to claims 1 to 4, **characterized in that** the implant comprises an outer osseous anchoring threading (15, 152) on a frustoconical portion whose flaring is reversed relative to the flaring formed by the truncated cone of the sleeve (2) in the deployed position.

6. The implant according to the preceding claims, **characterized in that** said expandable sleeve (2) includes longitudinal through-slots (24) extending up to its distal portion (23).

7. The implant according to the preceding claims, **characterized in that** the distal portion (23) of the sleeve is self-tapping thanks to the fact that it includes at least one notch or flute (231).

8. The implant according to claims 6 and 7, **characterized in that** there are as many self-tapping notches or flutes (231) as there are longitudinal through-slots (24).

9. The implant according to the preceding claims, **characterized in that** said expandable sleeve (2) includes longitudinal non-through slots (25).

10. The implant according to any of the preceding claims, **characterized in that** the screw (1) comprises at least one distance marker (16) to visualize the moment when the screwing of the screw (1) in the expandable sleeve (2) must be carried out in the opposite direction to the screwing the expandable sleeve (2) into the osseous tissue.

11. The implant according to the preceding claims, **characterized in that** said expandable sleeve (2) is made of plastic or elastic material.
